# EUROPEAN PATENT SPECIFICATION

(11) **EP 2 051 640 B1**
(45) Date of publication and mention of the grant of the patent: **20.01.2010**
(21) Application number: 07840996.8
(22) Date of filing: 16.08.2007
(51) Int. Cl.: A61B 17/00, A61B 17/04

(54) **SUTURING DEVICE**
NAHTVORRICHTUNG
DISPOSITIF DE SUTURE

(30) Priority: 16.08.2006 US 838124 P
(43) Date of publication of application: 29.04.2009
(73) Proprietor: Wilson-Cook Medical Inc., Winston-Salem, North Carolina 27105-4191 (US)
(72) Inventor: GIBBONS, JR., William, S., Winston-Salem, NC 27104 (US); SKERVEN, Gregory, J., Kernersville, NC 27284 (US)
(74) Representative: Garratt, Peter Douglas
(86) International application number: PCT/US2007/076085
(87) International publication number: WO 2008/022250

(56) References cited:
- WO-A-01/35833
- US-A- 5 431 666
- US-A1- 2006 142 784

## Description

### CROSS-REFERENCE TO RELATED APPLICATIONS

### BACKGROUND OF THE INVENTION

### 1. Field of the Invention

The present invention relates to medical devices and more particularly to endoscopic suturing devices for apposition of tissues.

### 2. Description of Related Art

There have been recent advancements of minimally-invasive surgical procedures. Such procedures have proven to be advantageous alternatives over prior invasive surgical procedures. Such advantages include quicker recovery time as well as more efficient hospital stays and medical costs.

U.S. Patent No. 5,431,666 (1995) describes a surgical apparatus for suturing body tissue which includes a handle assembly and an elongated body assembly detachably engaged to the handle assembly. The elongated body assembly includes an actuating mechanism for sequentially pulling first and second ferrule portions of a length of suture material through body tissue. The length of suture material and the ferrule portions are releasably retained in the elongated body assembly.

U.S. Patent Application Publication No. 2006/142784 (2006) discloses a device for remotely suturing an internal structure of a body. The device includes one or more anchors and means, operable from outside the body, for forcing the anchors out of the device. The anchors are located in or on the device so that they can be positioned adjacent the internal structure and when forced out of the device, enter and pass through the structure. The anchors are provided with sutures that can be tightened from outside the body to secure the anchors in place.

International Application Publication No. WO 01/35833 (2001) relates to a system for closure of a wound in a patient with a suture. A first apparatus includes a tissue engaging section coupled to a shaft, and first and second needles which extend from a housing through the shaft into the tissue engaging section. The tissue engaging section is directed through the wound, and has first and second gaps into which different sides of the wound can be received. A second apparatus secures a sleeve member over two ends of suture material in proximity of the wound and then cuts the suture material exiting the sleeve member.

Generally, endoscopic surgery involves incising through body walls, e.g., viewing or operating on ovaries, uterus, gall bladder, bowels, kidneys, and appendix, to name a few. Common endoscopic surgical procedures include arthroscopy, laparoscopy, and gastroentroscopy, to name a few. Although adequate, many devices and procedures may be improved. For example, there are a number of procedures that require a plurality of devices to complete a procedure.
For example, due the currently available apparatus, some endoscopic suturing procedures require a plurality of devices for completion of the procedures. Some of these devices are required to be placed at an angle to complete the procedure. In tum, more than one introduction of devices in a body vessel are undesirably required to complete the suturing procedures.

Thus, there is a need to provide a device and method of suturing that is simple and avoids multiple devices and, thus, multiple introductions thereof within a body vessel or cavity.

### BRIEF SUMMARY OF THE INVENTION

The present invention generally provides a suturing device that avoids a requirement of using multiple devices and multiple introductions of devices when suturing tissues. Embodiments of the present invention allow for a more efficient way of suturing tissues together and tying the suture wires. Embodiments of the present invention provide simple devices that have full thickness capability while providing "straight-on" placement of needles and sutures.

In one embodiment, the present invention provides a suturing device for apposition of tissues. The device comprises an introducer and first and second needle assemblies. The introducer includes a distal end and has a first port, a second port, and a suture port formed therethrough. The first needle assembly is disposed through the first port and a second needle assembly is disposed through the second port. The device further comprises a first ferrule and a second ferrule. The first ferrule is removably disposed in the first port at the distal end of the introducer, and is configured to receive the first needle assembly for tissue apposition. The second ferrule is removably disposed in the second port at the distal end of the introducer, and is configured to receive the second needle assembly for tissue apposition.

In this embodiment, the device further comprises a fastener for attaching suture wires. The fastener is disposed in the suture port at the distal end of the introducer. The device further comprises a first suture wire and a second suture wire. The first suture wire is attached to the first ferrule and extends through the fastener. The second suture wire is attached to the second ferrule and extends through the fastener for apposition of tissues.

In another example, the present invention provides a method of suturing a first tissue and a second tissue. The method comprises deploying a first ferrule through the first tissue. The first ferrule has a first suture wire attached thereto and extends through a fastener. The method further comprises deploying a second ferrule through the second tissue for apposition with the first tissue. The second ferrule has a second suture wire attached thereto and extends through the fastener. The method further comprises tensioning the first and second wires together to appose the first and second tissues together defining a tensioned position. The method further comprises fastening the first and second suture wires together in the tensioned position to maintain the first and second tissues in apposition.

Further objects, features, and advantages of the present invention will become apparent from consideration of the following description and the appended claims when taken in connection with the accompanying drawings.

### BRIEF DESCRIPTION OF THE DRAWINGS

Figure 1 is a perspective view of a suturing device for apposition of tissues in accordance with one embodiment of the present invention;

Figure 2 is a side view of the suturing device of Figure 1;

Figure 3 is an end view of the suturing device in accordance with one embodiment of the present invention;

Figure 4 is an enlarged side view of a distal end of the suturing device of Figure 2;

Figure 5 is an environmental view of a fastener for attaching suture wires from the suturing device in accordance with one embodiment of the present invention; and

Figure 6 is a flow chart of a method of suturing tissues in accordance with one example of the present invention.

### DETAILED DESCRIPTION OF THE INVENTION

The present invention generally provides a suturing device for apposition of tissues by straight-on and full thickness placement of the suturing device without requiring the use of additional apparatus. The suturing device comprises an introducer having needle assemblies that deploy ferrules through tissues. Each needle provides direct or straight-on placement of the ferrules in a time efficient manner. The ferrules are then tensioned and held together for apposition of the tissues.

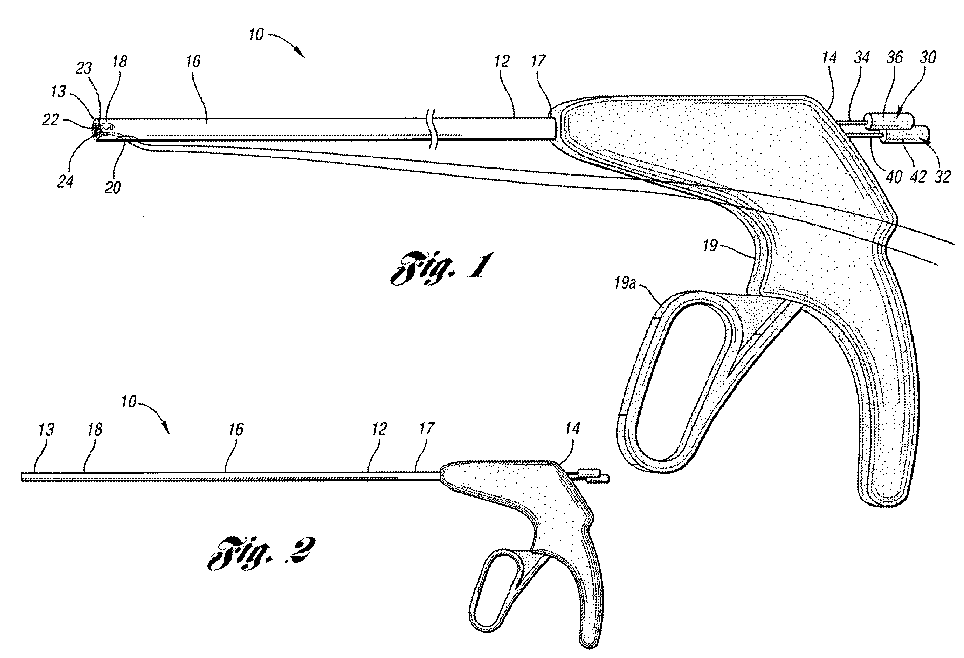

Figure 1 illustrates a suturing device 10 for apposition of tissues in accordance with one embodiment of the present invention. As shown, the suturing device 10 comprises an introducer 12 having a distal end 13 extending to a proximal end 14. In this embodiment, the introducer 12 comprises a tubular shaft 16 having proximal and distal portions 17,18 and a control handle 19 cooperable with and attached to the proximal portion 17 of the shaft 16. The control handle 19 may include a pivotable lever 19a. The tubular shaft 16 has a suture hole 20 longitudinally formed therethrough. As shown, the introducer 12 further includes a first port 22, a second port 23, and a suture port 24 all formed longitudinally through the proximal and distal portions 17,18 of the shaft 16.

Figures 1 and 2 illustrate the suturing device 10 further comprising a first needle assembly 30 and a second needle assembly 32. As shown, the first needle assembly 30 is disposed through the first port 22 and the second needle assembly 32 is disposed through the second port 23. In this embodiment, the first needle assembly 30 comprises a first elongate member 34 slidably disposed through the first port 22 and a first control knob 36 securedly attached to the proximal end of the first elongate member 34. As described in greater detail below, a clinician handles the first control knob 36 during the deployment of a ferrule for apposition of tissues. As such, the second needle assembly 32 comprises a second elongate member 40 slidably disposed through the second port 23 and a second control knob 42 securedly attached to the proximal end of the second elongate member 40. As described in greater detail below, a clinician handles the second control knob 42 in deploying a ferrule through a tissue for apposition.

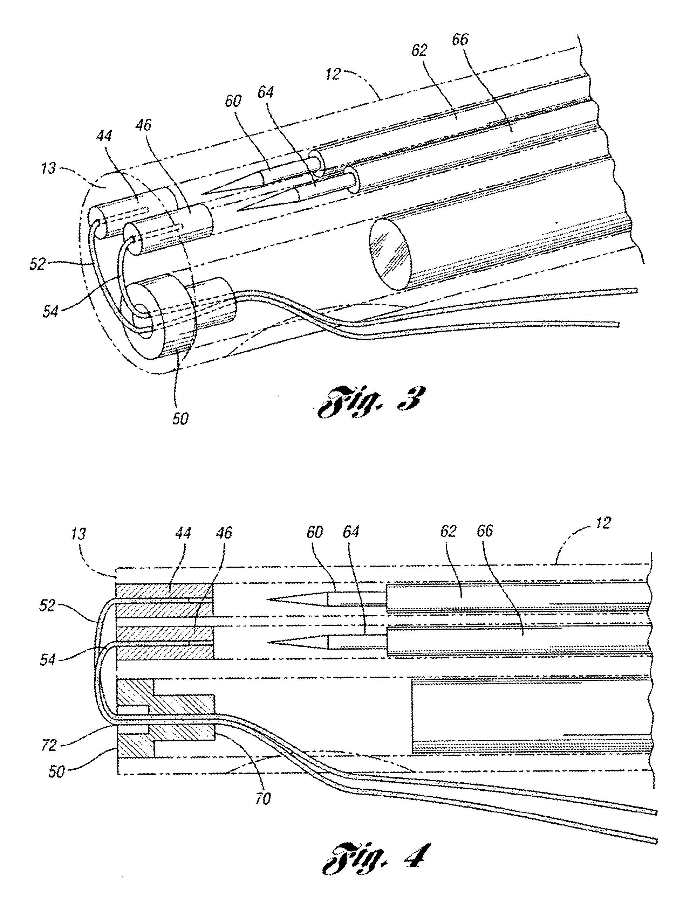

Figures 1 and 3 depict the distal end 13 of the introducer 12 carrying ferrules of the suturing device 10. As shown, the suturing device 10 further comprises a first ferrule 44 and a second ferrule 46 disposed at the distal end 13 of the introducer 12. In this embodiment, the first ferrule 44 is removably disposed in the first port 22 at the distal end 13 of the introducer 12 and the second ferrule 46 is removably disposed in the second port 23 at the distal end 13 thereof. Preferably, the first ferrule 44 is configured to receive the first needle assembly and the second ferrule 46 is configured to receive the second needle assembly 32 for the apposition of tissues. The first and second ferrules 44, 46 are removably lodged within their respective ports of the introducer 12 such that upon engagement of the needle assembly with the respective ferrule, there is enough friction or resistance to securedly mate and attach the needle assembly within the respective ferrule.

As shown in Figure 3, a fastener 50 for attaching suture wires (metallic or non-metallic) is disposed in the suture port 24 at the distal end of the introducer 12. In this embodiment, the fastener 50 is made of pinchable or crimpable material.
Any suitable crimpable material may be used, e.g, low density polymers, pure copper, stainless steel, crimplable metals, metal alloys, or high porousity materials.

Figure 3 further illustrates a first suture wire 52 (metallic or non-metallic) attached to the first ferrule 44 and extends through the fastener 50.
Preferably, the first suture wire 52 is attached at a mid-portion of the first ferrule 44.
From the fastener 50, the suture wire extends through the suture hole 20 of the introducer 12. Moreover, a second suture wire 54 (metallic or non-metallic) is attached to the second ferrule 46 and extends through the fastener 50. Preferably, the second suture wire 54 is attached at a mid-portion of the second ferrule 46.
From the fastener 50, the suture wire 52 proximally extends through the suture hole 20.

As shown, the first and second suture wires 52, 54 proximally extend passed the handle 19 so that a clinician may hold the wires and provide tension thereto. The clinician may retract or pull the wires 52,54 to appose the tissues together defining a tensioned position. The handle 19 is configured to crimp the fastener 50 in the tensioned position to hold the wires and appose the tissues together. This may be accomplished by any suitable manner. For example, the handle may include a component that is movable within the suture port of the introducer when the lever is pivotally squeezed against the handle. Upon squeezing of the lever, the component may engage the fastener at an angle to bend or crimp the fastener. The force from the component may also deploy the fastener adjacent the apposed tissues. Other manners of crimping and deploying the fastener may be implemented without falling beyond the scope the present invention.

Figure 4 shows the distal ends of the first and second needle assemblies 30, 32. The first needle assembly 30 is configured to be received by the first ferrule 44 and the second needle assembly 32 is configured to be received by the second ferrule 46 for tissue apposition. In this embodiment, the first needle assembly 30 comprises a first needle 60 configured to removably attach to the first ferrule 44. The assembly 30 further comprises a first tubular member 62 slidably disposed about the first needle 60 for releasing and deploying the first ferrule 44 from the first needle 60. As such, the second needle assembly 32 comprises a second needle 64 configured to removably attach to the second ferrule 46. The assembly 32 further comprises a second tubular member 66 slidably disposed about the second needle 64 for releasing and deploying the second ferrule 46 from the second needle 64.

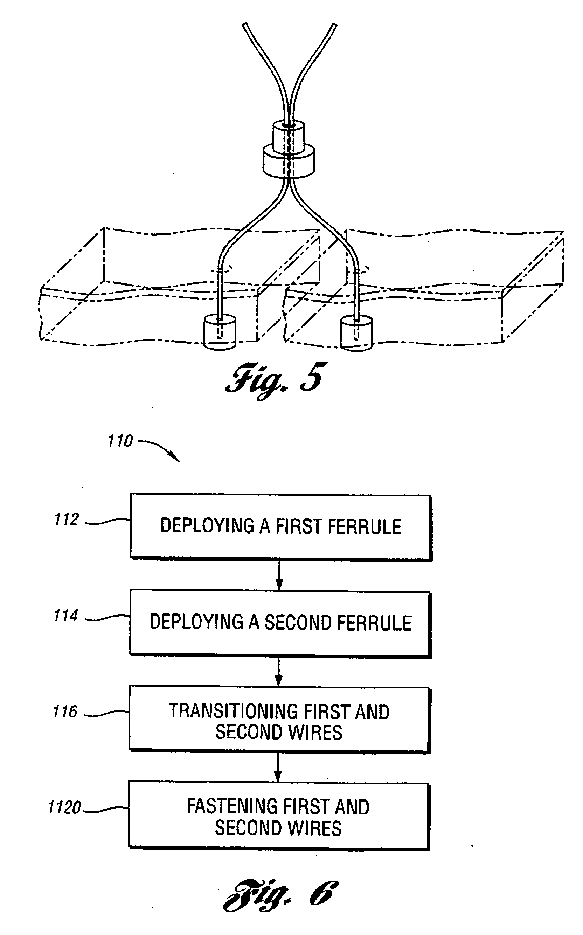

In use, the first needle 60 mates with the first ferrule 44 in the first port 22 of the introducer 12, and removably attaches within the first ferrule 44 to be introduced through a first tissue 80 for apposition (see Figure 5). Likewise, the second needle 64 mates with the second ferrule 46, and removably attaches to the second ferrule 64 in the second port 23 of the introducer 12. The second ferrule 46 is configured to be introduced through a second tissue 82 for apposition with the first tissue (see Figure 5). The first and second needle assemblies 30, 32 are slidably moved distally to engage and attach to the respective ferrules. The ferrules 44, 46 are introduced straight-on relative to the surface of the tissues, and through the full thicknesses thereof. Upon full thickness insertion of the ferrules through the tissues, each of the first and second tubular members 62, 66 slidably moves toward the respective ferrule to engage and push the ferrule, thereby disengaging the ferrule from the respective needle.

In this embodiment, the clinician moves first and second elongate members to engage and attach the first and second needles with the first and second ferrules, respectively. The first and second tubular members may slidably move by any suitable manner to disengage the ferrules from the respective needles. For example, the first and second control knobs may be in communication with the first and second tubular members. In this example, when squeezed or pressured, the knobs may be configured to slidably move the tubular member to disengage the ferrules from the respective needles. Other suitable way may be implemented without falling beyond the scope or spirit of the present invention.

In this embodiment, the fastener 50 is configured to hold the first and second suture wires 52, 54 together by crimping. One example of crimping the fastener is provided above. Preferably, the fastener 50 comprises distal and proximal openings 70, 72 formed therethrough. The first and second suture wires 52, 54 extend through the openings 70, 72. In this embodiment, the proximal opening 72 has a blade portion for trimming excess suture wires. After crimpling the fastener, the wires may be pulled to contact the blade portion and cut the excess suture wires.

Figure 6 depicts one method 110 of suturing a first tissue and a second tissue in accordance with one example of the present invention. As shown, the method comprises deploying a first ferrule through the first tissue in box 112. The first ferrule has a first suture wire attached thereto and extends through a fastener. This may be accomplished by the first needle assembly mentioned above. That is, the first needle assembly may engage with and attach to the first ferrule in the first port. The first needle assembly may then be slidably moved toward the first tissue for single insertion through the full thickness of the first tissue. This example provides a straight-on placement of the first ferrule through the first tissue.

The method further comprises deploying a second ferrule through the second tissue in box 114 for apposition with the first tissue. The second ferrule has a second suture wire attached thereto and extends through the fastener. The second needle assembly may place the second ferrule in the same manner as the first needle assembly and first ferrule described above.

As shown in Figure 5, the method further comprises tensioning the first and second wires together in box 116 to oppose the first and second tissues together defining a tensioned position. This may be accomplished by providing tension to the fastener as mentioned above. That is, sliding the fastener and proximally pulling the first and second suture wires provides tension between the wires and the tissues for apposition. As shown in Figure 5, the tension tightens the wires to appose of the first and second tissues.

Furthermore, the method further comprises fastening the first and second suture wires together in box 120 In the tensioned position to maintain the first and second tissues in apposition. This may be accomplished by crimping the fastener to hold and maintain the tensioned position of the first and second tissues in apposition as mentioned above. In this step, a first excess length of the suture wire and a second excess length of the suture wires extend from the fastener. The method further comprises cutting the excess lengths of the first and second suture wires. This may be accomplished by using the proximal opening of the fastener to cut the excess lengths of the first and second suture wires.

While the present invention has been described in terms of preferred embodiments, it will be understood, of course, that the invention is not limited thereto since modifications may be made to those skilled in the art, particularly in light of the foregoing teachings.

## Claims

1. A suturing device (10) for apposition of tissues, the device (10) comprising:
an introducer (12) with a distal end (13) and having a first port (22), a second port (23), and a suture port (24) formed therethrough;
a first needle (60) assembly (30) disposed through the first port (22) and a second needle (64) assembly (32) disposed through the second port (23);
a first ferrule (44) and a second ferrule (46), the first ferrule (44) removably disposed in the first port (22) at the distal end (13) of the introducer (12), the first ferrule (44) being configured to receive the first needle (60) assembly (30) for tissue apposition, the second ferrule (46) removably disposed in the second port (23) at the distal end (13) of the introducer (12), the second ferrule (46) being configured to receive the second needle (64) assembly (32) for tissue apposition;
a fastener (50) for attaching suture wires, the fastener (50) being disposed in the suture port (24) at the distal end (13) of the introducer (12); and
a first suture wire (52) and a second suture wire (54), the first suture wire (52) attached to the first ferrule (44) and extending through the fastener (50), the second suture wire (54) attached to the second ferrule (46) and extending through the fastener (50) for apposition of tissues.

2. The device (10) of claim 1 wherein the introducer (12) comprises:
a tubular shaft (16) having proximal and distal portions (17, 18), the shaft (16) having the distal end (13), the shaft (16) having the first port (22), the second port (23), and the suture port (24) formed therethrough; and
a control handle (19) cooperable with and attached to the proximal portion (17) of the shaft (16).

3. The device (10) of claim 1 wherein the first needle (60) assembly (30) comprises a first needle (60) attachable to the first ferrule (44) and a first tubular release (62) slidably disposed about the first needle (60) for releasing the first ferrule (44) from the first needle (60).

4. The device (10) of claim 1 wherein the second needle (64) assembly (32) comprises a second needle (64) attachable to the second ferrule (46) and a second tubular release (66) slidably disposed through the second needle (64) for releasing the second ferrule (46) from the second needle (64).

5. The device (10) of claim 1 wherein the fastener (50) attaches the first (52) and second suture wires (54) by crimping.

6. The device (10) of claim 1 wherein the first suture wire (52) is attached to a mid-portion of the first ferrule (44).

7. The device (10) of claim 1 wherein the second suture wire (54) is attached to a mid-portion of the second ferrule (46).

8. The device (10) of claim 2 wherein the tubular shaft (16) includes a suture hole (20) formed therethrough, the first and second suture wires (52, 54) extending through the fastener (50) and through the suture hole (20), exiting the tubular shaft (16), to a suture wire handle.

9. The device (10) of claim 1 wherein the fastener (50) comprises distal and proximal openings (70, 72) formed therethrough, the openings (70, 72) through which the first and second suture wires (52, 54) are disposed, the proximal opening (70) having a blade portion to trim excess suture wire.

## Patentansprüche

1. Nahtvorrichtung (10) zur Adaption von Geweben, wobei die Vorrichtung (10) Folgendes umfasst:
ein Einführungsinstrument (12) mit einem distalen Ende (13) und einer ersten Öffnung (22), einer zweiten Öffnung (23) und einer Nahtöffnung (24) durch es hindurch; eine erste Nadel- (60) Anordnung (30), die durch die erste Öffnung (22) verläuft, und eine zweite Nadel- (64) Anordnung (32, die durch die zweite Öffnung (23) verläuft; eine erste Zwinge (44) und eine zweite Zwinge (46), wobei die erste Zwinge (44) herausnehmbar in der ersten Öffnung (22) am distalen Ende (13) des Einführungsinstruments (12) angeordnet ist, wobei die erste Zwinge (44) so konfiguriert ist, dass sie die erste Nadel- (60) Anordnung (30) zur Adaption von Gewebe aufnehmen kann, wobei die zweite Zwinge (46) herausnehmbar in der zweiten Öffnung (23) am distalen Ende (13) des Einführungsinstruments (12) angeordnet ist, wobei die zweite Zwinge (46) so konfiguriert ist, dass sie die zweite Nadel- (64) Anordnung (32) zur Adaption von Gewebe aufnehmen kann; ein Befestigungselement (50) zur Befestigung von Nahtdrähten, wobei das Befestigungselement (50) in der Nahtöffnung (24) am distalen Ende (13) des Einführungsinstruments (12) angeordnet ist; und ein erster Nahtdraht (52) und ein zweiter Nahtdraht (54), wobei der erste Nahtdraht (52) an der ersten Zwinge (44) befestigt ist und durch das Befestigungselement (50) verläuft, wobei der zweite Nahtdraht (54) an der zweiten Zwinge (46) befestigt ist und zur Adaption von Gewebe durch das Befestigungselement verläuft.

2. Vorrichtung (10) nach Anspruch 1, worin das Einführungsinstrument (12) Folgendes umfasst: einen röhrenförmigen Schaft (16) mit proximalen und distalen Abschnitten (17, 18), wobei der Schaft (16) das distale Ende (13) aufweist, wobei durch den Schaft (16) die erste Öffnung (22), die zweite Öffnung (23) und die dritte Öffnung (24) geformt ist; und einen Kontrollgriff (19), der mit dem proximalen Abschnitt (17) des Schafts (16) zusammenarbeiten kann und auf diesem befestigt ist.

3. Vorrichtung (10) nach Anspruch 1, worin die erste Nadel- (60) Anordnung (30) eine erste Nadel (60), die an der ersten Zwinge (44) befestigt werden kann, und ein erstes röhrenförmiges Freigabeelement (62), das gleitend um die erste Nadel (60) befestigt ist, um die erste Zwinge (44) von der ersten Nadel (60) zu lösen, umfasst.

4. Vorrichtung (10) nach Anspruch 1, worin die zweite Nadel- (64) Anordnung (32) eine zweite Nadel (64) die an der zweiten Zwinge (46) befestigt werden kann, und ein zweites röhrenförmiges Freigabeelement (66), das gleitend durch die zweite Nadel (64) verläuft, um die zweite Zwinge (46) von der zweiten Nadel (64) zu lösen, umfasst.

5. Vorrichtung (10) nach Anspruch 1, worin das Befestigungselement (50) die ersten (52) und zweiten (54) Nahtdrähte durch Stauchen befestigt.

6. Vorrichtung (10) nach Anspruch 1, worin der erste Nahtdraht (52) an einem mittleren Abschnitt der ersten Zwinge (44) befestigt ist.

7. Vorrichtung (10) nach Anspruch 1, worin der zweite Nahtdraht (54) an einem mittleren Abschnitt der zweiten Zwinge (46) befestigt ist.

8. Vorrichtung (10) nach Anspruch 2, worin der röhrenförmige Schaft (16) ein durch ihn hindurch geformtes Nahtloch (20) aufweist, wobei die ersten und zweiten Nahtdrähte (52, 54) durch das Befestigungselement (50) und durch das Nahtloch (20) verlaufen und den röhrenförmigen Schaft (16) zu einem Nahtdrahtgriff verlassen.

9. Vorrichtung (10) nach Anspruch 1, worin das Befestigungselement (50) distale und proximale Öffnungen (70, 72) durch es hindurch umfasst, wobei durch die Öffnungen (70, 72) die die ersten und zweiten Nahtdrähte (52, 54) angeordnet sind, wobei die proximale Öffnung (70) einen Klingenteil zum Abschne4iden von überschüssigem Nahtdraht aufweist.

## Revendications

1. Dispositif de suture (10) pour l'apposition de tissus, le dispositif (10) comportant:
un introducteur (12) possédant une extrémité distale (13) et présentant un premier orifice (22), un deuxième orifice (23), et un orifice de suture (24) réalisés à travers celui-ci ;
un premier ensemble (30) d'aiguille (60) disposé dans le premier orifice (22) et un deuxième ensemble (32) d'aiguille (64) disposé dans le deuxième orifice (23) ;
une première virole (44) et une deuxième virole (46), la première virole (44) étant disposée de manière amovible dans le premier orifice (22) à l'extrémité distale (13) de l'introducteur (12), la première virole (44) étant conçue pour recevoir le premier ensemble (30) d'aiguille (60) pour l'apposition de tissus, la deuxième virole (46) étant disposée de manière amovible dans le deuxième orifice (23) à l'extrémité distale (13) de l'introducteur (12), la deuxième virole (46) étant conçue pour recevoir le deuxième ensemble (32) d'aiguille (64) pour l'apposition de tissus ;
une attache (50) pour attacher les fils de suture, l'attache (50) étant disposée dans l'orifice de suture (24) à l'extrémité distale (13) de l'introducteur (12) ; et
un premier fil de suture (52) et un deuxième fil de suture (54), le premier fil de suture (52) étant attaché à la première virole (44) et traversant l'attache (50), le deuxième fil de suture (54) étant attaché à la deuxième virole (46) et traversant l'attache (50) pour l'apposition de tissus.

2. Dispositif (10) selon la revendication 1 dans lequel l'introducteur (12) comporte:
une tige tubulaire (16) possédant des portions proximale et distale (17, 18), la tige (16) possédant l'extrémité distale (13), la tige (16) possédant le premier orifice (22), le deuxième orifice (23), et l'orifice de suture (24) formés à travers celle-ci ; et
une poignée de commande (19) coopérant avec la portion proximale (17) de la tige (16) et attachée à celle-ci.

3. Dispositif (10) selon la revendication 1 dans lequel le premier ensemble (30) d'aiguille (60) comporte une première aiguille (60) attachable à la première virole (44) et une première détente tubulaire (62) disposée de manière coulissante autour de la première aiguille (60) pour libérer la première virole (44) de la première aiguille (60).

4. Dispositif (10) selon la revendication 1 dans lequel le deuxième ensemble (32) d'aiguille (64) comporte une deuxième aiguille (64) attachable à la deuxième virole (46) et une deuxième détente tubulaire (66) disposée de manière coulissante à travers la deuxième aiguille (64) pour libérer la deuxième virole (46) de la deuxième aiguille (64).

5. Dispositif (10) selon la revendication 1 dans lequel l'attache (50) attache les premier (52) et deuxième (54) fils de suture par sertissage.

6. Dispositif (10) selon la revendication 1 dans lequel le premier fil de suture (52) est attaché à une portion médiane de la première virole (44).

7. Dispositif (10) selon la revendication 1 dans lequel le deuxième fil de suture (54) est attaché à une portion médiane de la deuxième virole (46).

8. Dispositif (10) selon la revendication 2 dans lequel la tige tubulaire (16) comprend un trou de suture (20) formé à travers celle-ci, les premier et deuxième fils de suture (52, 54) traversant l'attache (50) et le trou de suture (20), sortant de la tige tubulaire (16), vers une poignée de fil de suture.

9. Dispositif (10) selon la revendication 1 dans lequel l'attache (50) comporte des ouvertures distale et proximale (70, 72) formées à travers celle-ci, les ouvertures (70, 72) à travers lesquelles les premier et deuxième fils de suture (52, 54) sont disposés, l'ouverture proximale (70) possédant une portion de lame pour couper l'excédent de fil de suture.
